# EUROPÄISCHE PATENTSCHRIFT

(11) **EP 3 128 945 B1**
(45) Veröffentlichungstag und Bekanntmachung des Hinweises auf die Patenterteilung: **17.02.2021**
(21) Anmeldenummer: 15715254.7
(22) Anmeldetag: 09.04.2015
(51) Int. Cl.: A61C 13/00, A61C 13/01

(54) **VORGEFORMTER PROTHESENBASISROHLING**
PREFORMED PROSTHESIS-BASE BLANK
ÉBAUCHE DE BASE DE PROTHÈSE PRÉFORMÉE

(30) Priorität: 11.04.2014 DE 102014105189
(43) Veröffentlichungstag der Anmeldung: 15.02.2017
(73) Patentinhaber: Kulzer GmbH, 63450 Hanau (DE)
(72) Erfinder: SAVIC, Novica, 63526 Erlensee (DE); GALL, Silke Maren, 63755 Alzenau (DE); RENZ, Karl-Heinz, 63755 Alzenau (DE); STUMBAUM, Juliane, 80636 München (DE); BEUER, Florian, 83534 Freising (DE); SCHWEIGER, Josef, 83346 Bergen (DE)
(74) Vertreter: Schultheiss & Sterzel Patentanwälte PartG mbB
(86) Internationale Anmeldenummer: PCT/EP2015/057710
(87) Internationale Veröffentlichungsnummer: WO 2015/155284

(56) Entgegenhaltungen:
- EP-A1- 2 742 907
- WO-A1-2013/124452
- WO-A2-2013/068124
- US-A- 2 101 431

## Beschreibung

Die Offenbarung betrifft einen Prothesenbasisrohling zur Herstellung einer Prothesenbasis für eine Dentalprothese mit einem subtraktiven CAM-Verfahren, wobei der Prothesenbasisrohling aus einem Kunststoff oder aus einem Wachs besteht.

Die Erfindung betrifft ein Verfahren zur Herstellung einer Prothesenbasis für eine Dentalprothese gemäß den Ansprüchen 1-12. Offenbart ist weiter ein Set zur Durchführung mit einem solchen Prothesenbasisrohling.

Die Erfindung betrifft somit im Endeffekt die Herstellung von partiellen Kunststoffprothesen (Teilprothesen) und totale Kunststoffprothesen (Totalprothesen) für den zahnmedizinischen Bereich beziehungsweise von Prothesenbasen hierfür, die maschinell im CAM-Verfahren (CAM - Computer-Aided Manufacturing, Deutsch: rechner-unterstützte Fertigung) produziert werden. Bevorzugt werden die Dentalprothesen und die Prothesenbasen computergestützt mit CAD-Verfahren (CAD - Computer-Aided Design, Deutsch: rechnerunterstützte Konstruktion) konstruiert. Dabei wird zunächst eine Prothesenbasis hergestellt, die später auf dem zahnlosen oder bereichsweise zahnlosen Zahnfleisch des Kieferbogens eines Patienten aufliegt. Die Prothesenzähne werden anschließend individuell gefertigt und in die Prothesenbasis eingesetzt und dort befestigt. Die Prothesenbasis mit den aufgestellten Prothesenzähnen bildet dann die fertige Dentalprothese.

Der gängige Weg ist die analoge Erstellung von Dentalprothesen. Zur Herstellung der Prothesenbasis wird derzeit also meist ein analoges Verfahren verwendet, bei dem zunächst ein Abdruck des zahnlosen Kiefers des Patienten genommen wird. Aus diesem Abdruck wird eine Form gefertigt, die mit einem zahnfleischfarbenen Kunststoff ausgegossen wird. Nach Aushärtung des Kunststoffs wird dieser nachbearbeitet, um die gewünschte Form zu erhalten. Anschließend werden die separat hergestellten Prothesenzähne eingesetzt, die ebenfalls derzeit meist analog hergestellt werden. Zur Herstellung der Prothesenzähne werden diese manuell und einzeln auf einer Wachsbasis aufgestellt. Diese Wachsprothese wird im nächsten Schritt in einer Küvette mit Gips eingebettet, um dann nach Aushärten des Gipses die Wachsbasis mit heißem Wasser herauszuwaschen und einen Hohlraum für den Prothesenkunststoff zu schaffen. Die Prothesenzähne verbleiben dabei im Gips. Ein entsprechender Gips wird in den Hohlraum injiziert oder "gestopft" und man erhält nach Aushärtung des Kunststoffs die Prothese beziehungsweise den fertigen Prothesenzahn.

Die EP 2 742 907 A1 offenbart ein Verfahren zur Hersdtellung einer Dentalprothese, bei dem die durch Basalflächen der Prothesenbasis durchtretenden zervikalen Bereiche der Prothesenzähne entfernt werden. Bei der Aufstellung der konfektionierten Zähne werden diese der jeweiligen Mundsituation des Patienten vom Zahntechniker und gegebenenfalls auch vom Zahnarzt angepasst und beschliffen.

Ein solches Verfahren ist aus der WO 91/07141 A1 bekannt, bei dem eine Prothesenbasis auf der Basis eines Abdrucks aus einem Kunststoffblock gefräst wird. Es gibt bereits erste Verfahren, wie beispielsweise die aus der DE 10 2009 056 752 A1 oder der WO 2013 124 452 A1 bekannten Verfahren, bei denen die Teilbeziehungsweise Totalprothese digital aufgestellt und über CAD-CAM-Verfahren produziert wird. Die FR 2 582 932 A1 schlägt vor, dass eine Prothesenbasis mit Hilfe eine Wachsabdrucks und einer 3D-Kopierfräse erzeugt wird, dieses Verfahren ist aufwendiger als die modernen CAD-CAM-Verfahren. Aus der WO 2013 068 124 A2 ist ein Fräsblock mit vorgeformten Prothesenzähnen bekannt. Nachteilig ist hieran, dass die Prothesenzähne aus dem gleichen Material wie die Basis bestehen und bei einer Beschädigung das ganze Gebiss getauscht werden muss und die fertig konstruierte Prothese nur mit großem Aufwand an die Bedürfnisse des Patienten (beispielsweise bezüglich der Zahnfarbe oder der Okklusion und Zahnstellung) angepasst werden kann. Ein weiterer Nachteil liegt darin, dass die Basis und der Prothesenzahn (beziehungsweise die Prothesenzahnkrone) unterschiedliche Anforderungen haben aber aus dem gleichen Material gefertigt sind. So soll der Zahn vorrangig kauen und dabei unbeschädigt bleiben. Die Basis sollte hingegen die auftretenden Kräfte über die Schleimhaut verteilen. Dies kann bei der Verwendung nur eines Materials zu Problemen führen.

Aus der DE 20 2006 006 286 U1 ist ein runder Rohling zur Herstellung einer Zahnprothese bekannt. Dieser Rohling (der sogenannten Ronde) wird in eine CAM-Fräsmaschine eingespannt und auf Basis eines CAD-Modells automatisch abgefräst. Aktuelle Verfahren fräsen aus einem solchen vollen Block (der Ronde) eine lediglich 2 mm bis 3 mm dicke Prothesenbasis. Nachteile sind einerseits eine sehr hohe Materialverschwendung, die je nach anatomischer Situation des jeweiligen Patienten problemlos über 90% betragen kann. Das führt andererseits zu einer zeitintensiven Bearbeitungszeit durch die CAM-Vorrichtung, da sehr viel Material abgetragen werden muss. Darüber hinaus ist die Abnutzung der jeweiligen Fräswerkzeuge relativ hoch. Nachteilig ist hieran ferner, dass es relativ lange dauert, bis die Prothesenbasis hergestellt ist. Zudem nutzt sich durch die Bearbeitung des Rohlings der Fräskopf ab und muss in regelmäßigen Abständen erneuert werden. Das abgefräste Material (die Frässpäne) muss entsorgt oder recycelt werden.

Die Aufgabe der Erfindung besteht also darin, die Nachteile des Stands der Technik zu überwinden. Insbesondere soll ein Prothesenbasisrohling, ein Verfahren und ein Set bereitgestellt werden, mit dem eine möglichst einfache, vollständige und kostengünstige Bearbeitung des Prothesenbasisrohlings beziehungsweise eine möglichst einfache, vollständige und kostengünstige Herstellung der Prothesenbasis möglich ist. Der Fräskopf beziehungsweise das Werkzeug zum Durchführen des subtraktiven CAM-Verfahrens soll dabei möglichst wenig beansprucht werden. Zudem soll die Herstellung der Prothesenbasis möglichst schnell erfolgen können.

Die Offenbarung beschreibt einen Prothesenbasisrohling zur Herstellung einer Prothesenbasis für eine Dentalprothese mit einem subtraktiven CAM-Verfahren, wobei der Prothesenbasisrohling aus einem Kunststoff oder aus einem Wachs besteht, bei dem der Prothesenbasisrohling vorgeformt ist, wobei ein Zahnbogen oder ein Zahnbogenabschnitt als Materialverdickung des Prothesenbasisrohlings vorgeformt ist.

Dass der Zahnbogen oder Zahnbogenabschnitt vorgeformt ist, bedeutet, dass das Material für den zu erzeugenden Zahnbogen der Prothesenbasis oder den zu erzeugenden Zahnbogenabschnitt der Teil-Prothesenbasis bereits in der Materialverdickung enthalten ist. Dazu sind die Abmessungen des Zahnbogens oder des Zahnbogenabschnitts größer als eine Gruppe typischer zu fertigender Zahnbögen von Prothesenbasen einschließlich üblicher Toleranzen. Bevorzugt lassen sich mit einem erfindungsgemäßen Prothesenbasisrohling zwischen 5% und 100% aller realistisch zu erwartender Prothesenbasen herstellen.

Der Zahnbogen umfasst die Form der Prothesenbasis aber nicht der einzusetzenden Prothesenzähne. Nach Einsetzen der Prothesenzähne kann das mit dem Prothesenbasisrohling erzeugte Gebiss also höher sein, als die Verdickung des Zahnbogens des unbearbeiteten Prothesenbasisrohlings.

Mit der Erfindung wird auch vorgeschlagen, dass die Lage und Orientierung der zu erzeugenden Prothesenbasis in dem Prothesenbasisrohling durch die äußere Form des Prothesenbasisrohlings vorgegeben ist.

Selbstverständlich bleibt ein gewisser Spielraum, um unterschiedliche Prothesenbasen aus dem Prothesenbasisrohling fertigen zu können. Es reicht aus, wenn die Lage der zu erzeugenden Prothesenbasis in dem Prothesenbasisrohling auf 10 mm genau ist, bevorzugt auf 5 mm genau ist, und die Orientierung der zu erzeugenden Prothesenbasis in dem Prothesenbasisrohling auf 10° genau ist, bevorzugt auf 5° genau ist. Die Orientierung bezieht sich dabei auf mögliche Verkippungswinkel der zu erzeugenden Prothesenbasis relativ zum vorgeformten Prothesenbasisrohling.

Es kann vorgesehen sein, dass der Prothesenbasisrohling genau eine Symmetrieebene oder zwei senkrecht zueinander orientierte Symmetrieebenen aufweist, wobei genau eine Symmetrieebene bevorzugt ist. Bevorzugt kann dabei vorgesehen sein, dass die eine Symmetrieebene oder eine der Symmetrieebenen der Sagittalebene der zu erzeugenden Prothesenbasis entspricht.

Mit einer Weiterentwicklung wird vorgeschlagen, dass der Zahnbogen oder Zahnbogenabschnitt in okklusaler Richtung von der Auflagefläche auf dem Zahnfleisch weg mit abnehmender Breite geformt ist.

Hierdurch wird die Form des Prothesenbasisrohlings weiter an die Form von normalen beziehungsweise üblichen Prothesenbasen angepasst, die in okklusaler Richtung ebenfalls abnehmende Querschnitte senkrecht zur Verlauf des Zahnbogens aufweisen. Hierdurch wird die Menge des bei der Herstellung abzutragenden beziehungsweise abzufräsenden Materials weiter reduziert.

Es kann auch vorgesehen sein, dass der Prothesenbasisrohling auf der Seite zur Auflage auf dem Zahnfleisch eine gewölbte Vertiefung als Vorformung einer Aufnahme eines zahnlosen Kiefersattels oder eines Teils eines zahnlosen Kiefersattels aufweist, wobei die Vertiefung sich entlang des Zahnbogens oder Zahnbogenabschnitts erstreckt.

Auch diese Maßnahme dient dazu, die Form des Prothesenbasisrohlings weiter an die daraus zu erzeugende Form der Prothesenbasis anzupassen, um Material einzusparen und damit Zeit bei der Herstellung einzusparen und die Abnutzung des Fräswerkzeugs zu reduzieren.

Es wird auch vorgeschlagen, dass an wenigstens einer Seite des Prothesenbasisrohlings, bevorzugt an einer bukkalen Seite des Prothesenbasisrohlings, eine Halterung zur Befestigung des Prothesenbasisrohlings an einer CAM-Vorrichtung, insbesondere einer CAM-Fräsmaschine angeordnet ist und/oder eine Markierung zur Positionierung des Prothesenbasisrohlings vorgesehen ist.

Hierdurch kann sichergestellt werden, dass der Prothesenbasisrohling in der richtigen Orientierung und der richtigen Position in die CAM-Vorrichtung eingespannt werden kann.

Gemäß einer bevorzugten Ausführungsform wird auch vorgeschlagen, dass der Prothesenbasisrohling ausschließlich abgerundete Ecken und Kanten aufweist, bevorzugt keine Ecke oder Kante des Prothesenbasisrohlings einen Krümmungsradius von 0,5 mm, besonders bevorzugt von 2 mm unterschreitet. Bei Ausführungen des Prothesenbasisrohlings mit Halterung zur Befestigung des Prothesenbasisrohlings an der CAM-Vorrichtung, insbesondere der CAM-Fräsmaschine, kann vorgesehen sein, dass der Prothesenbasisrohling bis auf die Halterung ausschließlich abgerundete Ecken und Kanten aufweist, bevorzugt keine Ecke oder Kante des Prothesenbasisrohlings bis auf die Halterung einen Krümmungsradius von 0,5 mm, besonders bevorzugt von 2 mm unterschreitet.

Hierdurch kann verhindert werden, dass Ecken und Kanten des Prothesenbasisrohlings oder Teile davon bei der Bearbeitung durch das CAM-Verfahren abbrechen. Da die Halterung für die CAM-Vorrichtung nicht bearbeitet wird und später nur getrennt wird, können dort auch engere Krümmungsradien und Kanten realisiert werden.

Ferner kann vorgesehen sein, dass der Prothesenbasisrohling aus Polymethylmethacrylat (PMMA), Polyetherketon (PEK), Polyetheretherketon (PEEK), Polyamid (PA), Polycarbonat (PC) oder Polyurethan (PU) besteht.

Diese Materialien sind für die Bearbeitung des Prothesenbasisrohlings mit CAM-Verfahren besonders gut geeignet. Zudem lassen sich daraus auch ästhetisch passende Prothesenbasen fertigen.

Es kann auch vorgesehen sein, dass in dem Material, aus dem der Prothesenbasisrohling aufgebaut ist, anorganische Füllstoffe enthalten sind. Bevorzugt ist der Prothesenbasisrohling aus einem Kunststoff aufgebaut.

Als anorganische Füllstoffe kommen Siliziumoxide, Keramiken, Glaskeramiken oder deren Mischungen sowie anorganische Fasern aus Glas, Carbon oder organische Fasern aus Polyethylenen mit ultrahohem Molekulargewicht (UHMW-PE) in Frage. Die Füllstoffe können in Pulverform in dem Kunststoff verteilt sein. Bevorzugt kann der Kunststoff eine Transparenz aufweisen, die der Prothesenbasis ein natürliches Aussehen gibt.

Gemäß einer weiteren Ausführungsform kann vorgesehen sein, dass im Inneren des Zahnbogens oder des Zahnbogenabschnitts des Prothesenbasisrohlings und entlang des Zahnbogens oder des Zahnbogenabschnitts eine Versteifung des Materials des Prothesenbasisrohlings und/oder eine Armierung vorgesehen ist oder sind.

Hierdurch wird eine Verbesserung der mechanischen Eigenschaften der aus dem Prothesenbasisrohling erzeugten Prothesenbasis erzielt. Die mechanische Haltbarkeit einer derart erzeugten Dentalprothese ist im Vergleich zu herkömmlich mit CAD/CAM-Verfahren hergestellten Dentalprothesen verbessert.

Gemäß einer besonders bevorzugten Weiterbildung kann vorgesehen sein, dass der Zahnbogen als randseitige parabelförmige Verdickung des Materials des Prothesenbasisrohlings ausgebildet ist oder der Zahnbogenabschnitt als randseitige Verdickung des Materials in der Form eines Teilstücks einer Parabelform des Prothesenbasisrohlings ausgebildet ist.

Diese Form bietet die bestmögliche Ausgangsform zur Herstellung verschiedener Prothesenbasen. Die wesentliche Formgebung muss nämlich im Bereich dieser Zahnbögen durchgeführt werden. Gleichzeitig ist durch diesen Aufbau schon die grob die Form einer Prothesenbasis vorgegeben, so dass der Materialverlust beim Abtragen weiter minimiert werden kann.

Bei solchen Prothesenbasisrohlingen kann vorgesehen sein, dass die Verdickung eine Breite zwischen 8 mm und 30 mm aufweist und/oder die Verdickung eine Dicke beziehungsweise Höhe zwischen 5 mm und 30 mm aufweist. Die Breite und Dicke beziehungsweise Höhe der Verdickung entspricht dann der Breite und Dicke beziehungsweise der Höhe des Zahnbogens des Prothesenbasisrohlings.

Hierdurch ist genug Platz zum Einsetzen der Prothesenzähne in der Verdickung des Prothesenbasisrohlings, wenn dieser mit dem CAM-Verfahren bearbeitet ist.

Ferner kann bei diesen Prothesenbasisrohlingen vorgesehen sein, dass die Verdickung im Bereich um den Scheitelpunkt der parabelförmigen Verdickung eine größere Dicke beziehungsweise Höhe aufweist als an den Schenkeln der parabelförmigen Verdickung und/oder dass die Dicke beziehungsweise Höhe der Verdickung in Richtung des Scheitelpunkts des Parabelformabschnitts zunimmt.

Hierdurch kann eine weitere Anpassung der Form des Prothesenbasisrohlings an die zu erzeugende Prothesenbasis und damit eine Materialeinsparung erreicht werden. Die der vorliegenden Erfindung zugrundeliegenden Aufgaben werden gelöst durch ein Verfahren, entsprechend den Ansprüchen 1-12, zur Herstellung einer Prothesenbasis zur Herstellung einer Totaldentalprothese oder wenigstens einer Teildentalprothese aus einem Prothesenbasisrohling.

Aus dem Prothesenbasisrohling kann entweder eine Totalprothese für einen Kieferteil gefräst beziehungsweise geformt werden oder es können mehrere Teilprothesen aus einem Prothesenbasisrohling gefräst beziehungsweise geformt werden.

Mit dem erfindungsgemäßen Verfahren wird auch vorgeschlagen, dass der Prothesenbasisrohling aus einer Vielzahl von Prothesenbasisrohlingen ausgewählt wird, wobei die Auswahl rechnerisch auf der Grundlage des CAD-Modells der zu erzeugenden Prothesenbasis und den bekannten Abmessungen aller Prothesenbasisrohlinge erfolgt, wobei bevorzugt über eine Ausgabeeinrichtung eines Computers alle Prothesenbasisrohlinge der Auswahl vorgeschlagen werden und/oder über eine Eingabeeinrichtung ausgewählt werden können.

Hierdurch kann eine größere Anzahl unterschiedlicher Prothesenbasisrohlinge zur Verfügung gestellt werden, wobei jeder Prothesenbasisrohling schon besser an die Geometrie der zu erzeugenden Prothesenbasis angepasst ist, als wenn nur ein Prothesenbasisrohling oder wenige Prothesenbasisrohlinge zur Auswahl stünden.

Ferner kann erfindungsgemäß auch vorgesehen sein, dass der Prothesenbasisrohling ausgewählt wird oder die Prothesenbasisrohlinge ausgewählt werden aus der Menge der Prothesenbasisrohlinge, deren äußere Abmessungen die Form der zu erzeugenden Prothesenbasis, die durch das CAD-Modell berechnet ist, vollständig Aufnehmen können, wobei bevorzugt die vorgeschlagene Reihenfolge, insbesondere die angezeigte oder dargestellte Reihenfolge, der ausgewählten Prothesenbasisrohlinge bestimmt wird durch die Volumendifferenz zwischen der äußeren Form der Prothesenbasisrohlinge und dem CAD-Modell der zu erzeugenden Prothesenbasis.

Es kann erfindungsgemäß auch vorgesehen sein, dass automatisch der Prothesenbasisrohling vorgeschlagen wird oder verwendet wird, der die geringste Volumendifferenz zu dem CAD-Modell der zu erzeugenden Prothesenbasis aufweist.

Durch diese Maßnahmen gelingt es, bei einer gegebenen Auswahl verschiedener Prothesenbasisrohlinge denjenigen oder diejenigen auszuwählen, der am schnellsten bearbeitet werden kann oder die am schnellsten bearbeitet werden können, beziehungsweise bei denen am wenigsten Material abgetragen werden muss.

Es wird auch vorgeschlagen, dass bei der Berechnung des CAD-Modells der zu erzeugenden Prothesenbasis zuvor aufgenommene Daten zur Mundraumsituation des Patienten und/oder die äußere Form der Prothesenbasisrohlinge oder die äußere Form des Prothesenbasisrohlings berücksichtigt wird.

Hierdurch wird eine weitgehende Automatisierung des erfindungsgemäßen Verfahrens ermöglicht.

Es ist vorgesehen, dass mit dem CAM-Verfahren in dem vorgeformten Zahnbogen des Prothesenbasisrohlings mehrere Ausnehmungen zur Aufnahme von Prothesenzähnen in der hergestellten Prothesenbasis erzeugt werden, bevorzugt gefräst werden. Durch die Herstellung der Prothesenbasis und der Ausnehmungen in einem Schritt kann zum einen Zeit eingespart werden und zum anderen können Fehler vermieden werden, die beim erneuten beziehungsweise separaten Einspannen und Positionieren der halbfertigen Prothesenbasis in die selbe oder eine andere subtraktive CAM-Vorrichtung auftreten können.

Die Offenbarung beschreibt auch ein Set aufweisend eine Mehrzahl unterschiedlich geformter Prothesenbasisrohlinge und Daten zur äußeren Form des zumindest einen Prothesenbasisrohlings, bevorzugt aufweisend einen Datenträger auf dem die Daten gespeichert sind und das Set aufweisend Daten zur äußeren Form aller dieser Prothesenbasisrohlinge aufweist.

Der Erfindung liegt die überraschende Erkenntnis zugrunde, dass es mit den vorgeformten Prothesenbasisrohlingen gelingt, ein schnelleres und effizienteres Verfahren zur Herstellung von Prothesenbasen zu ermöglichen, bei denen die Werkzeuge der CAM-Vorrichtung geschont werden und der Materialverlust durch die subtraktiven CAM-Verfahren minimiert werden kann. Durch die geeignete Form bleiben die Prothesenbasisrohlinge dabei ausreichend flexibel, um problemlos an die unterschiedlichen Mundraumsituation und anderen Anforderungen für die zu erzeugende Prothesenbasis angepasst zu werden.

Die Prothesenbasisrohlinge, das Verfahren und das Set sind zur Bearbeitung mit CAD/CAM-Verfahren vorgesehen und hierfür besonders geeignet.

Der dieser Erfindung zu Grunde liegende Lösungsansatz geht bevorzugt von diversen vorgeformten Prothesenbasisrohlingen aus, die die oben genannten Nachteile dadurch eliminieren, dass es sich nicht um Vollkörper sondern um vorgeformte Rohlinge handelt. Die Erfindung zeichnet sich dadurch aus, dass sie - ähnlich Abformlöffeln - den menschlichen Kiefer anhand anatomischer Merkmale in Kategorien beziehungsweise Größen einteilt und dementsprechend weitestgehend vorgeformte Prothesenbasisrohlinge bereitstellt. Die in einem CAD-CAM-System verwendete Software wählt dann für die jeweilige Patientensituation den passenden Prothesenbasisrohling aus und übernimmt das sogenannte Nesting in den vorgeformten Prothesenbasisrohlingen (das best-mögliche Positionieren der virtuellen Form der zu erzeugenden Prothesenbasis in dem Prothesenbasisrohling). Die Prothesenbasisrohlinge können bevorzugt aus unterschiedlichen Materialien wie Kunststoffen oder Wachs hergestellt sein.

Die Vorteile der offenbarten Prothesenbasisrohlinge beziehungsweise des erfindungsgemäßen Verfahrens und des offenbarten Sets liegen unter anderem in den geringeren Fräszeiten, in einem geringeren Materialeinsatz an Prothesenbasismaterial und in einer Schonung der Fräswerkzeuge, das heißt in einem geringeren Verschleiß der Fräswerkzeuge im Vergleich zur Anwendung üblicher Fräsrohlinge.

Im Folgenden werden Ausführungsbeispiele der Erfindung anhand von vier schematisch dargestellten Figuren erläutert, ohne jedoch dabei die Erfindung zu beschränken. Dabei zeigt:
Figur 1: eine schematische perspektivische Ansicht eines Prothesenbasisrohlings;
Figur 2: eine schematische seitliche Ansicht eines Prothesenbasisrohlings;
Figur 3: eine schematische Aufsicht auf einen Prothesenbasisrohling; und
Figur 4: eine schematische Aufsicht auf einen Prothesenbasisrohling für eine Teilprothesenbasis.

In den Figuren werden auch bei unterschiedlichen Ausführungen für gleichartige Teile teilweise die gleichen Bezugszeichen verwendet.

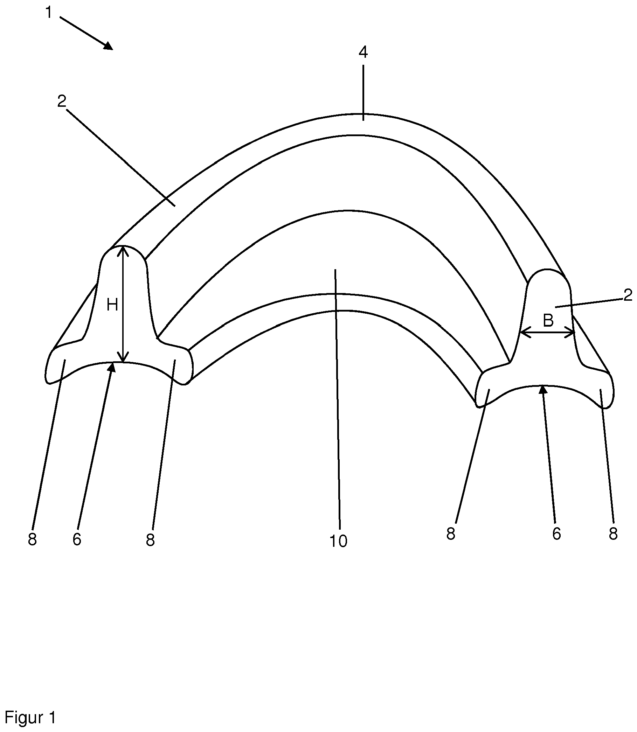

Figur 1 zeigt eine schematische perspektivische Ansicht eines Prothesenbasisrohlings 1. Der Prothesenbasisrohling 1 besteht aus einem Kunststoff, bevorzugt aus einem Polymer. Randseitig weist der Prothesenbasisrohling 1 eine parabellförmige Verdickung auf, die einen vorgeformten Zahnbogen 2 bildet. Der Prothesenbasisrohling 1 ist symmetrisch bezogen auf eine mittlere Ebene. Diese mittlere Ebene entspricht der Sagittalebene des Patienten, bei dem die mit dem Prothesenbasisrohling 1 hergestellte Dentalprothese eingesetzt wird. In der geometrischen Mitte, das heißt in der Sagittalebene, befindet sich der Scheitelpunkt 4 des parabelförmigen Zahnbogens 2. Der Scheitelpunkt 4 der daraus erzeugten Prothesenbasis wird später beim Patienten labial also lippenseitig angeordnet sein, wenn aus dem Prothesenbasisrohling 1 eine Totaldentalprothese gefertigt wird oder eine Teilprothese gefertigt wird, die zumindest die mittleren Schneidezähne umfasst. Auf der Seite, mit der die aus dem Prothesenbasisrohling 1 zu fertigende Prothesenbasis auf dem Zahnfleisch aufgelegt wird (in Figur 1 unten), ist eine Vertiefung 6 vorgesehen. Die Vertiefung 6 stellt eine Vorformung der Auflagevertiefung der zu erzeugenden Prothesenbasis dar, die das zahnlose Zahnfleisch des Patienten aufnehmen soll.

Seitlich neben dem Zahnbogen 2 sind Überstände 8 vorgesehen aus denen die kieferseitigen Verbindungsflächen der Prothesenbasis geformt werden. Dieser Überstand ist im Bereich des Scheitelpunkts 4 oral zu einem Mittelstück 10 verbreitert, aus dem eine palatinale, also gaumenseitige, oder unterhalb der Zunge im Mundraum des Unterkiefers aufliegende Anlagefläche formbar ist.

Der Zahnbogen 2 weist eine Breite B und eine Höhe H auf, wobei sich die Breite B und die Höhe H entlang des Zahnbogens ändern kann. Bei dem in Figur 1 dargestellten Prothesenbasisrohling 1 nimmt die Höhe H des Zahnbogens 2 zum Scheitelpunkt 4 hin zu. In diesem Bereich müssen, nach dem die Prothesenbasis herausgearbeitet wurde, die Schneidezähne beziehungsweise die Prothesen der Schneidezähne eingesetzt und befestigt werden. Die Breite B des Zahnbogens 4 wird so breit gewählt, dass eine ausreichende Variation der Ausformung der zu erzeugenden Prothesenbasis möglich ist.

Der gezeigte Prothesenbasisrohling 1 ist sowohl zur Herstellung einer Teilprothesenbasis oder mehrerer Teilprothesenbasen oder einer Totalprothesenbasis für einen Oberkiefer als auch für einen Unterkiefer geeignet. Es ist aber auch möglich, stärker vorgeformte Prothesenbasisrohlinge 1 vorzusehen, bei denen zwischen Oberkiefer und Unterkiefer unterschieden wird. Zudem oder alternativ können eine Vielzahl verschiedener Prothesenbasisrohlinge 1 vorgehalten werden oder als Set vorgesehen sein, die sich durch die Krümmung der Zahnbögen 2 und deren Breite unterscheiden und aus denen der passende Prothesenbasisrohling 1 ausgewählt werden kann. Die Prothesenbasisrohlinge 1 können sich auch durch unterschiedliche Höhen H und Breiten B der Zahnbögen 2 unterscheiden.

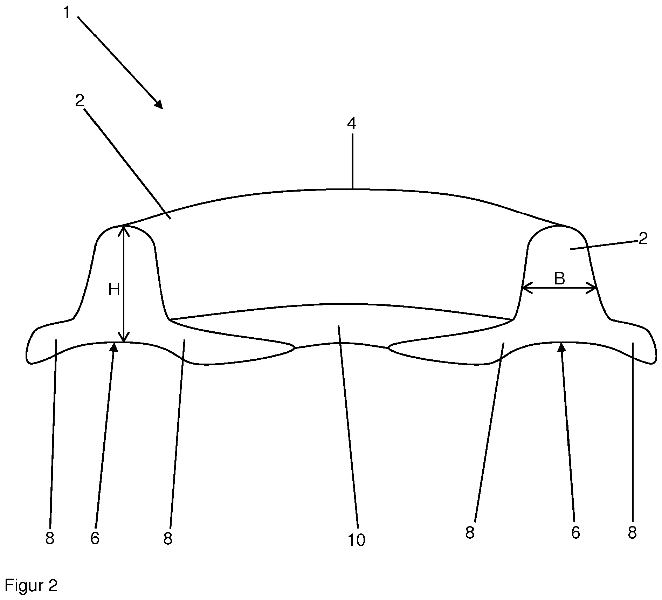

Figur 2 zeigt eine schematische seitliche Ansicht eines Prothesenbasisrohlings 1. Der Prothesenbasisrohling 1 weist, analog dem nach Figur 1, einen Zahnbogen 2 auf, der im Vergleich zu dem nach Figur 1 jedoch eine etwas größere Breite B aufweist. Der Zahnbogen 2 erstreckt sich randseitig in Parabelform entlang des Prothesenbasisrohlings 1. Im Bereich des Scheitelpunkts 4 des Prothesenbasisrohlings 1 ist die Höhe H des Zahnbogens 2 vergrößert.

An der Unterseite des Prothesenbasisrohlings 1 ist eine Vertiefung 6 vorgesehen (in Figur 2 unten), die als Vorformung einer Auflagefläche des zahnlosen Zahnfleischs des Patienten in der zu erzeugenden Prothesenbasis dient. Seitlich neben der Verdickung 2 durch den Zahnbogen 2 sind Verbreiterungen 8 beziehungsweise Überstände 8 vorgesehen, aus denen eine Verbindungsfläche zum Kiefersattel des Patienten geformt werden kann.

Das Mittelstück 10 reicht bei dieser Ausführungsform des Prothesenbasisrohlings 1 bis fast zur Mitte, so dass aus diesem Prothesenbasisrohling 1 auch eine großflächige Gaumenabdeckung geformt werden kann.

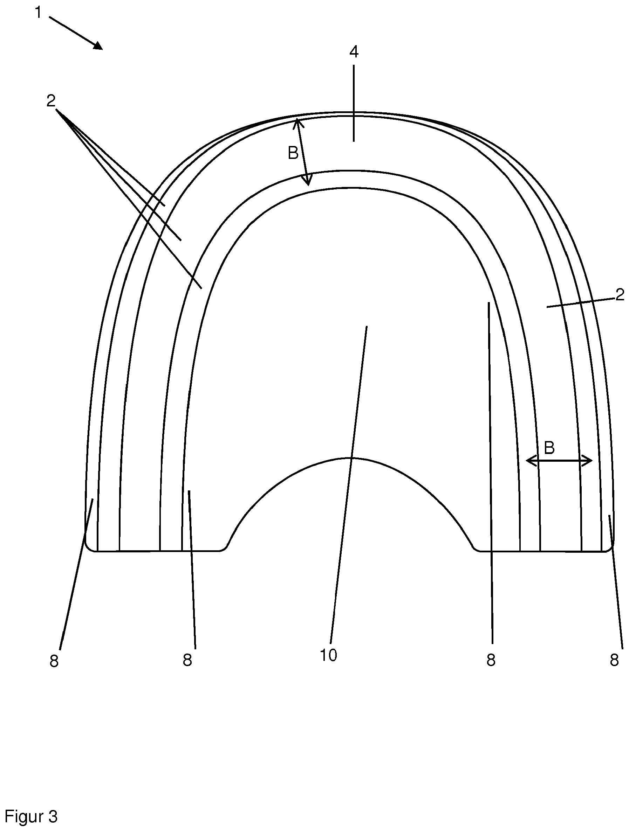

Figur 3 zeigt eine schematische Aufsicht auf einen weiteren Prothesenbasisrohling 1. Der Prothesenbasisrohling 1 weist abgerundete Ecken auf, um die Gefahr eines Abbrechens von Teilen des Prothesenbasisrohlings 1 bei der Bearbeitung zu verhindern.

Der Prothesenbasisrohling 1 weist eine randseitige parabelförmige Verdickung in Form eines Zahnbogens 2 auf, der sich analog der Ausführungen nach den Figuren 1 und 2 nach oben (in Figur 3 in Richtung des Betrachters) erstreckt. Im Bereich des Scheitelpunkts 4 des Zahnbogens 2 kann die Höhe des Zahnbogens 2 größer sein als an den gegenüberliegenden Seiten. Bei der Ausführung nach Figur 3 ist auch die Breite B im Bereich des Scheitelpunkts 4 erhöht.

Neben dem Zahnbogen 2 sind Überstände 8 vorgesehen, die dem gleichen Zweck dienen wie die Überstände 8 nach den Figuren 1 und 2. In der Mitte des Prothesenbasisrohlings 1 ist ein Mittelstück 10 vorgesehen, aus dem eine Gaumenplatte oder eine sublinguale Auflage der zu erzeugenden Prothesenbasis herstellbar ist. Im Bereich des Scheitelpunkts 4 des Prothesenbasisrohlings 1 ist der labiale Überstand 8 stark reduziert oder nicht mehr vorhanden. Auch der Querschnitt des Zahnbogens 2 senkrecht zur Parabelkurve beziehungsweise senkrecht zum Zahnbogen 2 verändert sich entlang des Zahnbogens 2. Die Steigung der labialen Flanke des Zahnbogens 2 ist im Bereich der Scheitelpunkts 4 reduziert.

Die Prothesenbasisrohlinge 1 nach den Figuren 1, 2 und 3 sind alle symmetrisch bezüglich der Sagittalebene aufgebaut, in der der Scheitelpunkt 4 liegt. Eventuell anatomisch notwendige Asymmetrien können beim Ausfräsen des Prothesenbasisrohlings 1 bei der Herstellung der Prothesenbasis erzeugt werden. Zur erfindungsgemäßen Herstellung einer Prothesenbasis wird ein Prothesenbasisrohlinge 1 aus einer Vielzahl von Prothesenbasisrohlingen 1 unterschiedlicher Abmessungen und Formen ausgewählt. Die äußeren Abmessungen der verschiedenen Prothesenbasisrohlinge 1 sind dazu elektronisch in einer Datenbank gespeichert. Die Mundraumsituation des zu behandelnden Patienten wird mit Hilfe eines 3D-Scanners eingescannt und digitalisiert. Anhand von diesen und gegebenenfalls von weiteren Daten wird ein virtuelles Modell der zu erzeugenden Prothesenbasis mit Hilfe eines CAD-Verfahrens erzeugt.

Anschließend wird getestet, in welchen oder in welche der vorhandenen Prothesenbasisrohlinge 1 die zu erzeugende Prothesenbasis passen könnte. Es wird eine Liste der möglichen Prothesenbasisrohlinge 1 auf einem Bildschirm eines Computers angeboten. Die Reihenfolge kann sich nach der Volumendifferenz zwischen der zu erzeugenden Prothesenbasis und dem Prothesenbasisrohling 1 richten, wobei geringere Volumendifferenzen favorisiert werden. Alternativ kann auch nur der Prothesenbasisrohling 1 mit der geringsten Volumendifferenz angezeigt werden oder nur die noch vorhandenen beziehungsweise eingelagerten Prothesenbasisrohlinge 1 berücksichtigt werden. Die geringe Volumendifferenz hat den Vorteil, dass bei diesen Prothesenbasisrohlingen 1 weniger Material abgetragen werden muss als bei Prothesenbasisrohlingen 1 mit einer höheren Volumendifferenz zu der zu erzeugenden Prothesenbasis.

Anschließend wird ein Prothesenbasisrohling 1 durch den Anwender oder vom Computer ausgewählt und ein Prothesenbasisrohling 1 dieses Typs in eine CAM-Vorrichtung, wie beispielsweise eine CAM-Vierachs-Fräse eingespannt. Dabei muss auf die richtige Orientierung des Prothesenbasisrohlings 1 in der CAM-Vorrichtung geachtet werden. Hierzu können an den Prothesenbasisrohlingen 1 Markierungen oder Ausformungen vorgesehen sein, die eine bestimmte Lage und Orientierung der Prothesenbasisrohlinge 1 in der CAM-Vorrichtung ermöglichen oder bevorzugt erzwingen.

Anschließend wird auf der Basis des CAD-Modells der zu erzeugenden Prothesenbasis diese aus dem eingespannten Prothesenbasisrohling 1 herausgefräst oder über ein anderes subtraktives CAM-Verfahren hergestellt.

In diese Prothesenbasis können auch gleich Ausnehmungen zur Aufnahme von Prothesenzähnen erzeugt werden. Anschließend werden Prothesenzähne in die Prothesenbasis eingeklebt und die Dental-Prothese ist fertiggestellt.

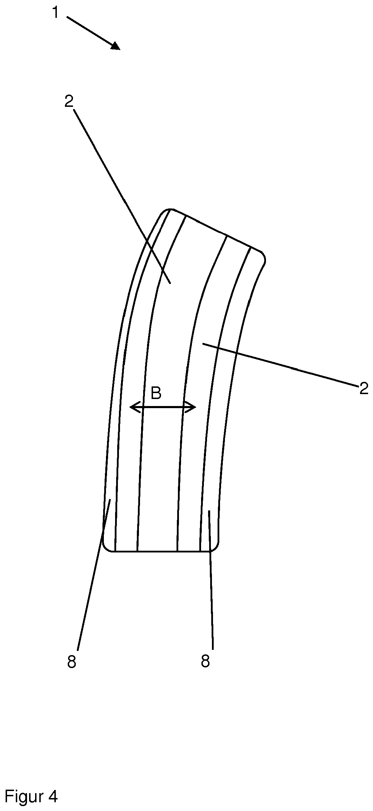

Figur 4 zeigt eine schematische Aufsicht auf einen Prothesenbasisrohling 21 für eine Teilprothesenbasis. Der Prothesenbasisrohling 21 stellt im Prinzip einen backenseitigen Teil des Prothesenbasisrohlings 1 nach Figur 3 dar. Ein Zahnbogenabschnitt 22 ist in Form eines Parabelabschnitts als Verdickung des Materials des Prothesenbasisrohlings 21 vorgesehen. Da die Breite B des Prothesenbasisrohlings 21 fast so breit ist, wie der gesamte Querschnitt des Prothesenbasisrohlings 21, ist der Zahnbogen 22 auch bei dieser Ausführungsform im Sinne der vorliegenden Erfindung randseitig angeordnet. Neben dem Zahnbogen 22 sind kurze Überstände 28 vorgesehen, die den gleichen Zweck erfüllen, wie die Überstände 8 der Ausführungsbeispiele nach den Figuren 1 und 2.

Auf der Unterseite des Prothesenbasisrohlings 21 ist eine Vertiefung vorgesehen (in der Aufsicht nach Figur 4 nicht zu sehen), die sich unterhalb des oberen Plateaus des Zahnbogens 22 in gleicher Richtung und Lage erstreckt. Die Vertiefung dient als Vorformung der späteren Auflagefläche auf das Zahnfleisch einer aus dem Prothesenbasisrohling 21 erzeugten Prothesenbasis beziehungsweise der daraus erzeugten Teil-Dentalprothese.

Das Herstellungsverfahren für den Prothesenbasisrohling 21 nach Figur 4 läuft analog dem zu den Figuren 1 bis 3 geschilderten Verfahren ab, wobei eben nur eine Teil-Dentalprothese erzeugt wird.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 21: Prothesenbasisrohling
- 2: Vorgeformter Zahnbogen
- 4: Scheitelpunkt des Zahnbogens
- 6: Vertiefung an der Auflagefläche zum Zahnfleisch
- 8, 28: Überstand
- 10: Mittelstück
- 22: Vorgeformter Zahnbogenabschnitt
- B: Breite des Zahnbogens
- H: Höhe des Zahnbogens

## Patentansprüche

1. Verfahren zur Herstellung einer Prothesenbasis zur Herstellung einer Totaldentalprothese oder wenigstens einer Teildentalprothese mit einem Prothesenbasisrohling (1, 21), wobei der Prothesenbasisrohling (1, 21) aus einem Kunststoff oder aus einem Wachs besteht, und wobei jeweils ein Zahnbogen (2) oder ein Zahnbogenabschnitt (22) als Materialverdickung des Prothesenbasisrohlings (1, 21) vorgeformt ist, wobei der Zahnbogen (2) oder der Zahnbogenabschnitt (22) das Material für den zu erzeugenden Zahnbogen der Prothesenbasis oder den zu erzeugenden Zahnbogenabschnitt der Teil-Prothesenbasis bereits in der Materialverdickung enthält, das Verfahren aufweisend die folgenden Verfahrensschritte:
1) Befestigen des Prothesenbasisrohlings (1, 21) in einer CAM-Vorrichtung zum Abtragen von Material des Prothesenbasisrohlings (1, 21) mit einem CAM-Verfahren und
2) Abtragen von Material des Prothesenbasisrohlings (1, 21) mit der CAM-Vorrichtung auf der Basis eines berechneten CAD-Modells, wobei in dem vorgeformten Zahnbogen (2) des Prothesenbasisrohlings (1, 21) mehrere Ausnehmungen zur Aufnahme von Prothesenzähnen in der hergestellten Prothesenbasis erzeugt werden.

2. Verfahren nach Anspruch 1, **dadurch gekennzeichnet, dass** der Prothesenbasisrohling (1, 21) aus einer Vielzahl von Prothesenbasisrohlingen (1, 21) ausgewählt wird, wobei die Auswahl rechnerisch auf der Grundlage des CAD-Modells der zu erzeugenden Prothesenbasis und den bekannten Abmessungen aller Prothesenbasisrohlinge (1, 21) erfolgt.

3. Verfahren nach Anspruch 2, **dadurch gekennzeichnet, dass** über eine Ausgabeeinrichtung eines Computers alle Prothesenbasisrohlinge (1, 21) der Auswahl vorgeschlagen werden und/oder über eine Eingabeeinrichtung ausgewählt werden können.

4. Verfahren nach Anspruch 2 oder 3, **dadurch gekennzeichnet, dass** der Prothesenbasisrohling (1, 21) ausgewählt wird oder die Prothesenbasisrohlinge (1, 21) ausgewählt werden aus der Menge der Prothesenbasisrohlinge (1, 21), deren äußere Abmessungen die Form der zu erzeugenden Prothesenbasis, die durch das CAD-Modell berechnet ist, vollständig Aufnehmen können.

5. Verfahren nach Anspruch 4, **dadurch gekennzeichnet, dass** die vorgeschlagene Reihenfolge, insbesondere die angezeigte oder dargestellte Reihenfolge, der ausgewählten Prothesenbasisrohlinge (1, 21) bestimmt wird durch die Volumendifferenz zwischen der äußeren Form der Prothesenbasisrohlinge (1, 21) und dem CAD-Modell der zu erzeugenden Prothesenbasis.

6. Verfahren nach Anspruch 4 oder 5, **dadurch gekennzeichnet, dass** automatisch der Prothesenbasisrohling (1, 21) vorgeschlagen wird oder verwendet wird, der die geringste Volumendifferenz zu dem CAD-Modell der zu erzeugenden Prothesenbasis aufweist.

7. Verfahren nach einem der vorangehenden Ansprüche, **dadurch gekennzeichnet, dass**
bei der Berechnung des CAD-Modells der zu erzeugenden Prothesenbasis zuvor aufgenommene Daten zur Mundraumsituation des Patienten berücksichtigt werden.

8. Verfahren nach einem der vorangehenden Ansprüche, **dadurch gekennzeichnet, dass**
bei der Berechnung des CAD-Modells der zu erzeugenden Prothesenbasis die äußere Form der Prothesenbasisrohlinge (1, 21) oder die äußere Form des Prothesenbasisrohlings (1, 21) berücksichtigt wird.

9. Verfahren nach einem der vorangehenden Ansprüche, **dadurch gekennzeichnet, dass**
aus dem Prothesenbasisrohling (1, 21) eine Totalprothese für einen Kieferteil gefräst oder geformt wird oder mehrere Teilprothesen aus einem Prothesenbasisrohling (1, 21) gefräst beziehungsweise geformt werden.

10. Verfahren nach einem der vorangehenden Ansprüche, **dadurch gekennzeichnet, dass**
die Lage und Orientierung der zu erzeugenden Prothesenbasis in dem Prothesenbasisrohling (1, 21) durch die äußere Form des Prothesenbasisrohlings (1, 21) vorgegeben wird.

11. Verfahren nach einem der vorangehenden Ansprüche, **dadurch gekennzeichnet, dass**
der Prothesenbasisrohling (1, 21) aus Polymethylmethacrylat, Polyetherketon, Polyetheretherketon, Polyamid, Polycarbonat, Polyurethan oder Wachs besteht.

12. Verfahren nach einem der vorangehenden Ansprüche, **dadurch gekennzeichnet, dass**
der Zahnbogen (2) oder der Zahnbogenabschnitt (22) die Form der Prothesenbasis aber nicht der einzusetzenden Prothesenzähne umfasst.

## Claims

1. A method for the production of a denture base for the production of a total denture or at least a partial denture with a denture base blank (1, 21), wherein the denture base blank (1, 21) consists of a plastic material or a wax, and wherein a dental arch (2) or a dental arch section (22) is pre-formed as a material thickening of the denture base blank (1, 21), wherein the dental arch (2) or the dental arch section (22) contains the material for the denture arch to be produced of the denture base or the denture arch section to be produced of the partial denture base in the material thickening, the method comprising the following method steps:
1) Affixing of the denture base blank (1, 21) in a CAM device for the removal of material of the denture base blank (1, 21) using a CAM method, and
2) Removal of material from the denture base blank (1,21) with the CAM device on the basis of a calculated CAD model, wherein several recesses for holding prosthetic teeth are created in the pre-formed dental arch (2) of the denture base blank (1, 21) in the denture base produced.

2. The method according to claim 1, **characterized in that**
the denture base blank (1,21) is selected from a plurality of denture base blanks (1, 21), wherein the selection is made using a calculation based on the CAD model of the denture base to be produced and the known dimensions of all denture base blanks (1,21).

3. The method according to claim 2, **characterized in that**
by means of an output facility of a computer, all denture base blanks (1, 21) from the selection can be recommended and/or can be selected via an input facility.

4. The method according to claim 2 or 3, **characterized in that**
the denture base blank (1,21) is selected or the denture base blanks (1, 21) are selected from the quantity of denture base blanks (1, 21) the outer dimensions of which can fully hold the form of the denture base to be produced, which is calculated by the CAD model.

5. The method according to claim 4, **characterized in that**
the recommended sequence, in particular the displayed or shown sequence, of the selected denture base blanks (1, 21) is determined by the volume difference between the outer form of the denture base blanks (1, 21) and the CAD model of the denture base to be produced.

6. The method according to claim 4 or 5, **characterized in that** the denture base blank (1, 21) is automatically recommended or used which comprises the lowest volume difference to the CAD model of the denture base to be produced.

7. The method according to any one of the preceding claims, **characterized in that** when calculating the CAD model of the denture base to be produced, data previously recorded relating to the oral cavity situation of the patient is taken into account.

8. The method according to any one of the preceding claims, **characterized in that** when calculating the CAD model of the denture base to be produced, the outer form of the denture base blanks (1, 21) or the outer form of the denture base blank (1, 21) is taken into account.

9. The method according to any one of the preceding claims, **characterized in that** from the denture base blank (1, 21) either a total denture is milled or formed for part of a jaw, or several partial dentures are milled or formed from a denture base blank (1, 21).

10. The method according to any one of the preceding claims, **characterized in that** the position and orientation of the denture base to be produced in the denture base blank (1, 21) is pre-specified by the outer form of the denture base blank (1,21).

11. The method according to any one of the preceding claims, **characterized in that** the denture base blank (1, 21) consists of polymethyl methacrylate, polyether ketone, polyether ether ketone, polyamide, polycarbonate or polyurethane or wax.

12. The method according to any one of the preceding claims, **characterized in that** dental arch (2) or the dental arch section (22) comprises the form of the denture base, but does not comprise the form of prosthetic teeth to be inserted.

## Revendications

1. Procédé de fabrication d'une base de prothèse permettant la fabrication d'une prothèse dentaire totale ou d'au moins une prothèse dentaire partielle avec une ébauche de base de prothèse (1, 21), dans lequel l'ébauche de base de prothèse (1, 21) est constituée d'une matière plastique ou d'une cire, et dans lequel une arcade dentaire (2) ou un segment d'arcade dentaire (22) sont respectivement préformés sous forme d'un épaississement de matière de l'ébauche de base de prothèse (1, 21), dans lequel l'arcade dentaire (2) ou le segment d'arcade dentaire (22) contiennent déjà le matériau pour l'arcade dentaire de la base de prothèse à créer ou le segment d'arcade dentaire de la base de prothèse partielle à créer dans l'épaississement de matière, le procédé présentant les étapes de procédé suivantes :
1) la fixation de l'ébauche de base de prothèse (1, 21) dans un dispositif de FAO pour le retrait de matière de l'ébauche de base de prothèse (1, 21) avec un procédé FAO, et
2) le retrait de matière de l'ébauche de base de prothèse (1, 21) avec le dispositif de FAO en se basant sur un modèle de CAO calculé, dans lequel, plusieurs cavités sont créées dans l'arcade dentaire (2) préformée de l'ébauche de base de prothèse (1, 21) pour l'admission de dents prothétiques dans la base de prothèse fabriquée.

2. Procédé selon la revendication 1, **caractérisé en ce que**
l'ébauche de base de prothèse (1, 21) est choisie à partir d'un grand nombre d'ébauches de base de prothèse (1, 21), dans lequel la sélection a lieu par calcul sur la base du modèle de CAO de la base de prothèse à créer et des dimensions connues de toutes les ébauches de base de prothèse (1, 21).

3. Procédé selon la revendication 2, **caractérisé en ce que**
toutes les ébauches de base de prothèse (1, 21) de la sélection sont proposées par le biais d'un dispositif d'édition d'un ordinateur et/ou peuvent être choisies par le biais d'un dispositif de saisie.

4. Procédé selon la revendication 2 ou la revendication 3, **caractérisé en ce que** l'ébauche de base de prothèse (1, 21) est choisie, ou les ébauches de base de prothèse (1, 21) sont choisies à partir de la multitude d'ébauches de bases de prothèse (1, 21) dont les dimensions extérieures peuvent totalement prendre la forme de la base de prothèse à créer, qui est calculée par le modèle de CAO.

5. Procédé selon la revendication 4, **caractérisé en ce que** le classement proposé, notamment le classement indiqué ou représenté des ébauches de base de prothèse (1, 21) sélectionnées est déterminée par la différence de volume entre la forme extérieure des ébauches de base de prothèse (1, 21) et le modèle de CAO de la base de prothèse à créer.

6. Procédé selon la revendication 4 ou la revendication 5, **caractérisé en ce que** l'ébauche de base de prothèse (1, 21) qui présente la différence de volume la plus faible par rapport au modèle de CAO de la base de prothèse à créer, est proposée ou est employée automatiquement.

7. Procédé selon l'une des revendications précédentes, **caractérisé en ce que** l'on tient compte de données concernant la situation de l'espace buccal du patient précédemment enregistrées, lors du calcul du modèle de CAO de la base de prothèse à créer.

8. Procédé selon l'une des revendications précédentes, **caractérisé en ce que** l'on tient compte de la forme extérieure des ébauches de base de prothèse (1, 21) ou de la forme extérieure de l'ébauche de base de prothèse (1, 21) lors du calcul du modèle de CAO de la base de prothèse à créer.

9. Procédé selon l'une des revendications précédentes, **caractérisé en ce qu'**une prothèse totale pour une partie de mâchoire est fraisée ou est façonnée à partir de l'ébauche de base de prothèse (1, 21) ou plusieurs prothèses partielles sont fraisées, respectivement, façonnées à partir d'une ébauche de base de prothèse (1, 21).

10. Procédé selon l'une des revendications précédentes, **caractérisé en ce que** la position et l'orientation de la base de prothèse à créer est prédéfinie dans l'ébauche de base de prothèse (1, 21) par la forme extérieure de l'ébauche de base de prothèse (1, 21).

11. Procédé selon l'une des revendications précédentes, **caractérisé en ce que** l'ébauche de base de prothèse (1, 21) est constituée de polyméthyl méthacrylate, d'une polyéther cétone, d'une polyéther éther cétone, de polyamide, de polycarbonate, de polyuréthane ou de cire.

12. Procédé selon l'une des revendications précédentes, **caractérisé en ce que** l'arcade dentaire (2) ou le segment d'arcade dentaire (22) comprend la forme de la base de prothèse, mais non les dents prothétiques à implanter.
